# EUROPÄISCHE PATENTSCHRIFT

(11) **EP 3 761 919 B1**
(45) Veröffentlichungstag und Bekanntmachung des Hinweises auf die Patenterteilung: **27.04.2022**
(21) Anmeldenummer: 19709710.8
(22) Anmeldetag: 07.03.2019
(51) Int. Cl.: A61F 5/01, A61F 5/02, A61F 5/30

(54) **MOBILISIERENDE HÜFTORTHESE**
MOBILIZATION HIP ORTHOSIS
ORTHÈSE DE LA HANCHE MOBILISATRICE

(30) Priorität: 07.03.2018 DE 102018203443
(43) Veröffentlichungstag der Anmeldung: 13.01.2021
(73) Patentinhaber: Bauerfeind AG, 07937 Zeulenroda-Triebes (DE)
(72) Erfinder: WIEDNER, Juliane, 07937 Zeulenroda-Triebes (DE); HESS, Heinrich, 66271 Kleinblittersdorf (DE); BAUERFEIND, Hans B., 07937 Zeulenroda-Triebes (DE)
(74) Vertreter: Wohlfahrt, Jan Günther
(86) Internationale Anmeldenummer: PCT/EP2019/055704
(87) Internationale Veröffentlichungsnummer: WO 2019/170806

(56) Entgegenhaltungen:
- CN-A- 107 106 318
- DE-A1-102014 226 841
- US-A1- 2009 326 427
- US-A1- 2013 178 775
- US-A1- 2013 283 492
- US-A1- 2014 066 973

## Beschreibung

Diese Erfindung betrifft eine verbesserte Hüftgelenkorthese (Coxarthrose-Orthese) sowie einen Beckengurt mit neuartiger Pelottenanordnung wie in den Ansprüchen definiert. Die Hüftgelenkorthese (Coxarthrose-Orthese) sowie der Beckengurt eignen sich für die Therapie und postoperative Rehabilitation von insbesondere degenerativen Hüftgelenkserkrankungen. Coxarthrose-Orthesen sind Orthesen zur Stützung oder Führung der Gelenkbewegung des Hüftgelenks. Eine Fassung oberhalb, das heißt proximal des zu stützenden Hüftgelenks ist in Form eines fest an der Hüfte anliegenden Beckengurts vorgesehen. Eine Fassung unterhalb, das heißt distal des Hüftgelenks ist in Form einer Oberschenkelfassung vorgesehen. Mit dem Beckengurt fest verbunden ist der obere (proximale) Abschnitt oder Arm dieser hüftgelenküberbrückenden Gelenkschiene, die Oberschenkelfassung ist mit dem unteren (distalen) Abschnitt oder Arm der Gelenkschiene fest verbunden. Die Gelenkschiene besitzt ein gegebenenfalls aus der primären Schwenkebene kippbares Axialgelenk, welches in der Schwenkbewegung dem Hüftgelenk folgt und das Hüftgelenk dadurch führt und entlastet.

Nachteilig bei solchen bekannten Hüftorthesen ist, dass wegen der für die gewünschte Stütz- und Führungswirkung notwendigen mechanisch stabilen Konstruktion Vergurtungen und Bandagenelemente notwendig sind, die insbesondere an dem Beckengürtel stark in das darunterliegende Weichteilgewebe des Patienten eindrücken. Da aber Patienten mit Hüftgelenkbeschwerden wie die degenerative Coxarthrose gerade in diesem Körperbereich, das heißt besonders in den beteiligten Muskelpartien schmerzempfindlich sind, leidet der Tragekomfort der Orthese im Gebrauch. Es zeigt sich, dass Patienten daher solche fest anliegenden Orthesen-Fassungen nicht gut akzeptieren, (geringe Compliance). Eine erhöhte Schmerzempfindlichkeit oder gar dauerhafte Schmerzen entstehen insbesondere in Folge chronischer Entzündung der Gelenkweichteile und der beteiligten Muskeln. Bekanntermaßen werden dadurch beteiligte Muskelpartien kontrakt, das heißt, es bilden sich dort lokale Verhärtungen in aus. Diese Muskelschmerzen können auf Bein- und Rückenpartien, besonders auf die tiefe Rückenmuskulatur, ausstrahlen und sind für den Patienten sehr belastend. Durch die dauerhafte unspezifische Druckbelastung dieser Muskelpartien bei fest angelegtem Beckengurt können sich die Reizzustände an solchen lokalen Verhärtungen sogar noch verstärken. Es wird beobachtet, dass solche Patienten gerade bei angelegter Orthese eine Schonhaltung einnehmen, die mit geringer oder unphysiologischer Bewegung einhergeht und in Folge die Therapiewirkung der Orthese reduziert. Gleichzeitig verstärkt auch die Schonhaltung Beschwerden und Schmerzen in benachbarten oder gar entfernteren Körperregionen, insbesondere die tiefen Rückenschmerzen, unter welchen viele Hüftgelenkspatienten zusätzlich leiden.

Problematisch sind somit neben Gelenkschmerzen, welche unmittelbar von dem arthrotischen oder anderweitig geschädigten Hüftgelenk ausgehen, und von sich aus bereits aufgrund der entzündlichen Kontraktur der Gelenkkapsel zu einer Bewegungseinschränkung führen, die zusätzlichen Muskelschmerzen, welche durch schmerzhafte Kontraktur der beteiligten Muskeln hervorgerufen werden. Besonders die Muskelschmerzen aber werden, wie vorstehend ausgeführt, durch bekannte Gelenkorthesen nicht gemildert, sondern im Gegenteil eher verstärkt.

Für eine zielführende Therapie ist daher regelmäßig eine physikalische Therapie, vor allem die sogenannte Extensionsbehandlung, erforderlich. Dort wird das Bein an dem betroffenen Hüftgelenk einer Zugbelastung ausgesetzt, was die Gelenkkapsel entlastet und in Reaktion die Kontrakturen in Gelenk und Muskel reduzieren, Schmerzen lindern und die Beweglichkeit verbessern kann. Für eine effizientere Extensionsbehandlung ist in der physikalischen Therapie auch die gezielte Massage der Muskulatur oberhalb des Gelenks vorgesehen, was hilft, die kontrakte Muskulatur zu detonisieren. Die behandelten Muskeln werden nachgiebiger und die Extensionsbehandlung am Gelenk somit effizienter.

Die DE 10 2014 226 841 A1 offenbart eine Pelotte für eine Bandage oder Orthese, wobei die Pelotte eine vorderseitige Oberfläche und eine rückseitige Oberfläche aufweist, wobei die rückseitige Oberfläche zumindest in einem Teilbereich Stege aufweist, die eine Gitternetzstruktur mit Zwischenräumen ausbilden.

Die CN 107 106 318 A offenbart eine Rückenpelotte für eine Bandage oder Orthese, wobei die Rückenpelotte eine vorderseitige Oberfläche und eine rückseitige Oberfläche aufweist, wobei die rückseitige Oberfläche zumindest in einem Teilbereich Stege aufweist, die eine Gitternetzstruktur mit Zwischenräumen ausbilden, wobei die vorderseitige Oberfläche Erhebungen aufweist und wobei die Rückenpelotte einstückig und aus einem einzigen Material gebildet ist.

Die US 2013/283492 A1 offenbart ein individuelles Ausrüstungsaufhängsystem mit aktiver Unterstützung für den Lendenbereich, umfassend einen Gürtel, der die Taille des Benutzers umgibt und einen Rahmen, der auf dem Gürtel mit einer elastischen Membran aus synthetischem elastomerem kristallinem Material getragen wird, das auf den Rahmen angebracht ist, um zwischen dem Gürtel und dem Benutzer angeordnet zu werden.

Es ist wünschenswert, bekannte Hüftorthesen derart zu verbessern, dass sie neben einer ausreichend stützenden und gelenkführenden orthetischen Funktion zusätzlich eine schmerzlindernde Wirkung auf die betroffenen Bereiche des Hüftgelenks und damit verbundener Körperabschnitte ausüben. Das der vorliegenden Erfindung zugrundeliegende technische Problem bestand daher insbesondere in der Bereitstellung einer verbesserten Hüftgelenkorthese, deren Tragekomfort und damit deren Compliance verbessert ist, und die darüber hinaus ein erweitertes Anwendungsspektrum für die Therapie besitzt und in der Nachsorge und Rehabilitation eingesetzt werden kann, wobei über die rein gelenkstützende Funktion hinaus eine therapieunterstützende Zusatzwirkung erreicht werden kann. Besonders soll dabei eine Orthese bereitgestellt werden, welche auch die tiefen Rückenschmerzen, welche oft von diesen kontrakten Muskelpartien des Hüftgelenks ausgehen, reduzieren kann.

Das technische Problem wird gelöst durch einen neuartigen Beckengurt, der bei Hüftgelenkbeschwerden als Orthese getragen werden kann und darüber hinaus als Beckenfassung, das heißt als wesentlicher Bestandteil einer Coxarthrose-Gelenkorthese eingesetzt werden kann. Der erfindungsgemäße Beckengurt weist dazu besonders eine spezifische Anordnung von Kissen und Pelotten auf. Es wird gemäß eines ersten Aspekts ein Beckengurt bereitgestellt, der für sich genommen als Orthese zur Unterstützung von therapeutischen Maßnahmen bei Hüftgelenkleiden dienen kann. Gemäß eines zweiten Aspekts dieser Erfindung kann der Beckengurt aber auch Bestandteil einer Coxarthrose-Gelenkorthese sein und dort die obere (proximale) Beckenfassung bildet, von der aus die Gelenkschiene zu einer distalen Oberschenkelfassung führt.

Die Erfindung stellt dazu in dem ersten Aspekt den Gegenstand des Anspruchs 1 bereit, das heißt besonders einen Beckengurt, der geeignet ist für eine oder als Bestandteil einer Coxarthrose-Orthese, wobei dieser Beckengurt neben einem Beckengurtgrundkörper und zwei zum Anlegen des Beckengurts um die Hüfte eines Patienten verbindbaren seitlichen Beckengurtenden eine spezifische Anordnung von Pelotten aufweist. Erfindungsgemäß ist der Beckengurt besonders dadurch gekennzeichnet, dass an dessen Innenseite, im Bereich mindestens eines Beckengurtendes mindestens ein Noppenkissen ausgebildet oder angebracht ist, welches mindestens eine vorspringende, insbesondere elastische, Noppe aufweist. Erfindungsgemäß ist dabei vorgesehen, dass diese mindestens eine Noppe über das Noppenkissen an dem Beckengurt an der Innenseite so positioniert ist, dass diese Noppe im angelegten Zustand des Beckengurts, insbesondere zwangsweise, zu dem Mittleren Gesäßmuskel (Musculus gluteus medius) des Patienten hin gerichtet ist, das heißt besonders an diesem anliegt.

Erfindungsgemäß ist an dem Beckengurt, ein, insbesondere elastisches, Kissen mit insbesondere elastischer von dem Kissen vorspringenden Noppe ausgebildet, welche im angelegten Zustand dieses Beckengurts unmittelbar in die Weichteilregion des Patienten oberhalb, das heißt proximal, des Hüftgelenks eingreift und dort insbesondere im Bereich des Mittleren Gesäßmuskels in das Muskelgewebe drückt, sobald der Beckengurt bestimmungsgemäß und fest an der Hüfte des Patienten angelegt wird. Gegenüber bekannten Polsterungen von Becken- oder Hüftgurten, welche primär der Abpolsterung von knochigen Vorsprüngen, insbesondere des Beckenkamms dienen sollen, wirkt das erfindungsgemäß vorgesehene Noppenkissen mit der mindestens einen vorstehenden Noppe aufgrund ihrer spezifischen Positionierung in der Höhe des Muskelbauches des Mittleren Gesäßmuskels. Die, insbesondere elastisch bewegliche, Noppe wirkt bei Bewegung des Patienten, insbesondere beim Gehen, krafteinleitend auf gezielte Bereiche des Mittleren Gesäßmuskels, und zwar in einer alternierenden, intermittierenden und damit massierenden Weise. Dabei zeigt sich überraschend, dass diese Krafteinleitung und damit verbundene Massagewirkung nicht durch eine aktive Bewegung der Noppe erreicht wird. Vielmehr wird durch die Eigenbewegung des Muskelbauchs in der Bewegung, beim Gehen bewirkt, dass die, insbesondere elastisch nachgiebige Noppe passiv auf den Muskel einwirkt. Der Muskel schiebt sich sozusagen in seiner Bewegung selbst auf die und entlang der Noppe, welche an der Oberfläche des Muskels entlang gleitet und aufgrund der Normalkraft durch den gespannt angelegten Gurt an Ort und Stelle gehalten wird und sich, je nach Position des Muskels, bevorzugt elastisch, abkippt oder aufstellt Durch die so erzeugbare "Selbstmassage" des Muskels lassen sich die dort nachteiligerweise vorhandenen kontrakten Bereiche des Muskels aktiv detonisieren. Muskelverkürzung und -verhärtung sowie Schmerzen können reduziert und der Widerstand des Muskels gegen eine extendierende Wirkung reduziert werden.

Die Extension von Hüftgelenk und Muskel findet einerseits bereits durch die Bewegung, das heißt beim Gehen statt, da der entsprechend detonisierte Muskel nun nachgiebiger ist und eine höhere Beweglichkeit erreicht wird. Zum anderen kann eine zusätzliche externe Extensionsbehandlung, insbesondere im Rahmen einer physikalischen Therapie, durch die Wirkung der erfindungsgemäßen Orthese vorbereitet und unterstützt werden, da die Extensionsbehandlung auf einen bereits durch die Noppen des Beckengurts detonisierten Muskel trifft und dadurch entsprechend effektiv wird.

Für die beabsichtige Detonisierung des Mittleren Gesäßmuskels sind an dem Noppenkissen mindestens eine vorstehende Noppe, bevorzugt aber mindestens zwei oder mehr Noppen, die zueinander beabstandet angeordnet sind, ausgebildet. In besonderen Varianten sind drei Noppen, in anderen Varianten vier Noppen vorgesehen. Die Noppen sind in Form und mechanischer Eigenschaft bevorzugt spezifisch so ausgebildet, dass sie an sogenannten "Triggerpunkten" des Mittleren Gesäßmuskels angreifen können. Vorliegend werden unter "Triggerpunkte" einzelne lokale Bereiche an oder in einem Muskel verstanden, welche kontrakt, das heißt verhärtet und verkürzt sind und welche ursächlich für Schmerzen des Patienten sind. Triggerpunkte entstehen durch entzündliche Vorgänge, aber auch durch lokale Überlastung von Muskelfasern. Die kontrakten Muskelfasern an den Triggerpunkten können, insbesondere durch lokale Übersäuerung und Sauerstoffmangel, selbst lokale Entzündungsreaktionen auslösen, was in Folge andere Körperbereiche in Mitleidenschaft zieht (myofasziales Schmerzsyndrom). Entzündungsmarker und Wachstumsfaktoren können in diesen Muskelbereichen nachgewiesen werden. Durch gezielten Druck insbesondere durch die massierende Bewegung der unter der Normalkraft fest und unter Druck anliegenden Noppe spezifisch auf den oder die Triggerpunkte kann die lokale Verhärtung gelöst und der Muskel so insgesamt detonisiert werden. Das betroffene Muskelareal wird dabei gedehnt, die Verkrampfung gelöst, und akkumulierte entzündungsauslösende Stoffe werden abgeführt.

Um eine individuelle Anpassung der Wirkung der Noppen des Noppenkissens an den Zustand des mittleren Gesäßmuskels des jeweiligen Patienten zu ermöglichen, ist bevorzugt vorgesehen, dass das Noppenkissen ortsveränderlich an dem Beckengurtgrundkörper, entweder direkt oder über eine Trägerplatte, fixierbar ist. Alternativ oder zusätzlich ist vorgesehen, dass verschieden gestaltete Noppenkissen zur Auswahl stehen, welche eine, zwei, drei oder vier Noppen aufweisen, die mehr oder weniger weit voneinander beabstandet sind. Bei der Konfektionierung der Orthese kann auf die Erkenntnisse aus der manuellen Therapie von Triggerpunkten zurückgegriffen werden. Triggerpunkte können als schmerzhafte lokale Verhärtungen an dem Muskel ertastet werden. Dort können die Noppen des erfindungsgemäßen Beckengurts platziert werden.

Zur Erhaltung und Verbesserung der Wirkung des oder der Noppen, ist das Noppenkissen zusammen mit der mindestens einen Noppe bevorzugt einstückig ausgebildet. Alternativ ist die mindestens eine Noppe, vorzugsweise mehrere Noppen, in das Noppenkissen eingesetzt. In diesem Fall ist es möglich, durch geeignete Materialwahl die Beweglichkeit der Noppe an dem Noppenkissen, das heißt besonders das Abkippen der Noppe, unabhängig von der Resilienz der einzelnen Noppe gegenüber dem Muskelgewebe, worauf die Noppe einwirkt, einzustellen.

Bevorzugt sind Noppenkissen und/oder Noppen aus einem Elastomermaterial, besonders aus Silikonelastomer, vorzugsweise RTV-Silikonkautschuk oder alternativ aus PU-Elastomer gebildet. Ohne an die Theorie gebunden sein zu wollen, ist bei der Auswahl des Elastizitätsmoduls (Shore A-Härte) der einzelnen Komponenten bevorzugt, dass die Resilienz des Elastomermaterials der Noppen beziehungsweise des Noppenkissens geringer ist als diejenige des Haut- und Fettgewebes zwischen Noppen und Oberfläche des Mittleren Gesäßmuskels, aber bevorzugt höher als diejenige des angespannten und insbesondere verhärteten Muskelgewebes. Das heißt bevorzugt sind die Noppen weniger nachgiebig als das Fettgewebe, so dass sie die Kraft- und Druckeinleitung auf den darunter liegenden Muskel bewirken können, und sie sind bevorzugt nachgiebiger als das verhärtete Muskelgewebe, um eine schmerzfreie und sanfte Massage des Muskelgewebes zu ermöglichen, was erfindungsgemäß, wie vorstehend erläutert, besonders dadurch erzielt wird, dass sich der Muskel selbst beim Bewegen, das heißt besonders beim Gehen, an den Noppen vorbei bewegt und die Noppen auslenkt, wodurch die Noppen das Muskelgewebe massieren. Bevorzugt beträgt die Shore A-Härte von Noppenkissen und davon abstehenden Noppen von 40 bis 60.

In einer bevorzugten Ausführung ist das Noppenkissen flach und in Längserstreckung in Form eines Kreisbogens oder "bananenförmig" ausgebildet. Sind mehrere Noppen an dem Noppenkissen ausgebildet, sind diese, bevorzugt gleichmäßig beabstandet, an der gedachten Kreisbogen Linie entlang angeordnet. In einer alternativen Ausführung ist das Noppenkissen oval oder kreisförmig und trägt insbesondere zwei bzw. eine Noppe. Das Noppenkissen besitzt in Längserstreckung bevorzugt eine Länge von etwa 5 bis 15 cm und ist dabei bevorzugt etwa 2 bis 5 cm breit. Die Dicke des Noppenkissens beträgt bevorzugt etwa von 1 bis 3 cm. Die von dem Noppenkissen abstehenden Noppen sind kreisförmig bis kreissektorförmig ausgebildet und weisen etwa einen Durchmesser von bevorzugt 10 bis 30 mm auf. Die Noppen ragen dabei bevorzugt etwa 3 bis 15 mm, besonders bevorzugt etwa 6 mm über die Oberfläche des Noppenkissens hinaus. Es ist vorgesehen, das je nach Therapieziel, Anwendungsgebiet und/oder anatomischer Größe des Patienten eine passend dimensioniertes Noppenkissen und/oder eine passend strukturierte Noppenanordnung aus einer Reihe von vorkonfektionierten unterschiedlich dimensionierten und strukturierten Noppenkissen ausgewählt werden kann, um die Orthese spezifisch daran anzupassen.

Erfindungsgemäß bevorzugt sind die Noppe oder Noppen an ihrer zu dem Muskel weisenden Oberseite feinstrukturiert, und dazu bevorzugt mit einer oder mehreren schmalen Längsrippen versehen. Bevorzugt sind Rillen oder Rippen, die im Wesentlichen parallel zu dem Muskelfaserverlauf der angesprochenen Muskelregion orientiert sind. Die Rillen oder Rippen der feinstrukturierten Noppenoberseite weisen jeweils eine Breite von bevorzugt etwa 1 bis 3 mm auf. Die feinstrukturierte Noppenoberseite legt sich bei angelegter Orthese entlang der Längsfasern des Muskels, besonders des Mittleren Gesäßmuskels, an. Dies erlaubt ein tieferes Eindringen der durch die Noppen vermittelten massierenden Krafteinleitung in den Muskel. Muskelbegleitende Gewebe wie venöse und lymphatische Kapillaren werden dadurch besser erreicht und werden direkt stimuliert. Es zeigt sich, dass so ein besserer Abtransport von entzündungsauslösenden Stoffen und Stoffwechselprodukten aus den kontrakten Bereichen des Muskels und auch eine verbesserte Versorgung erreicht werden kann. In einer Variante davon sind, alternativ oder zusätzlich zu Längsstrukturen, kleine einzeln bewegliche halbkugelförmige oder prismatische oder pyramidale Erhebungen vorgesehen, welche sich vor allem bei Bewegung des Muskels und Druck der Noppe auf die Muskelregion zwischen die Muskelfasern schieben.

Das Noppenkissen kann zusammen mit den Noppen in einer Tasche, beispielsweise aus Frotteematerial, eingenäht sein oder mit einer hautschonenden Beschichtung oder Beflockung versehen sein.

Besonders ist vorgesehen, dass das Noppenkissen an dem Beckengurtgrundkörper direkt fixiert ist. In einer alternativen und bevorzugten Variante weist der Beckengurtgrundkörper mindestens eine starre Trägerplatte auf, worauf das Noppenkissen fixierbar ist, insbesondere ortsveränderlich fixierbar und/oder abnehmbar ist. Dazu ist bevorzugt vorgesehen, an dem Noppenkissen an sich bekannte mechanische Verkopplungselemente, besonders Klettverschlusselemente, vorzusehen, welche in entsprechende Klettverschlussgegenelemente an der Trägerplatte eingreifen können.

In einer besonderen Variante ist vorgesehen, dass die Trägerplatte an dem Beckengurtgrundkörper mit einer Gelenkplatte des proximalen Arms einer Gelenkschiene, welche das Hüftgelenk überbrückt, fixierbar ist, um den Beckengurtgrundkörper mit der Gelenkschiene zu verbinden, um mit dem erfindungsgemäßen Beckengurt eine Coxarthrose-Gelenkorthese zu bilden. Dabei ist besonders vorgesehen, dass die Trägerplatte als Tasche an dem Beckengurtgrundkörper ausgebildet ist, worin die Gelenkplatte des proximalen Arms der Gelenkschiene aufnimmt. Alternativ kann die Gelenkplatte an der Trägerplatte mit anderen an sich bekannten Verkopplungsmitteln wie Nieten, Knöpfe oder Klettverschlusselementen fixiert werden.

In bevorzugter Ausführung sind an der Orthese, beispielsweise unmittelbar an dem erfindungsgemäßen Beckengurt oder alternativ und zusätzlich an der Gelenkschiene, welche zur Ausbildung einer Coxarthrose-Gelenksorthese mit diesem Beckengurt verbindbar ist, spezifisch weitere Strukturen ausgebildet, welche eine aktive Extension an dem Hüftgelenk unterstützen. Dazu ist in einer ersten Variante eine distale Aktivpelotte vorgesehen, welche unterhalb, das heißt distal zu dem Angriffspunkt der mindestens einen Noppe des Noppenkissens lokalisiert ist, und zwar im angelegten Zustand des Beckengurts besonders unmittelbar im Bereich der Gelenkkapsel am Becken, und dort besonders in die distal zu dem Hüftgelenk liegende Region des Mittleren Gesäßmuskels aktiv druckeinleitend eingreifen kann.

Unter einer "Aktivpelotte" wird im Zusammenhang mit der Erfindung ein insbesondere kleineres, härteres Pelottenkissen verstanden, das an der Orthese fixiert ist und passiv oder insbesondere aktiv und von der Bewegung an dem Noppenkissen unabhängig bewegbar ist.

In einer ersten Ausführung ist das Noppenkissen zu einer distalen Aktivpelotte an dem Beckengurt benachbart angeordnet. In einer Variante davon sind distale Aktivpelotte und Noppenkissen gemeinsam an dem Beckengurt angeordnet und dort insbesondere fixiert. In einer alternativen Variante ist das Noppenkissen an dem Beckengurt beziehungsweise Beckengurtgrundkörper fixiert und die distale Aktivpelotte ist in einer Gelenkplatte der das Hüftgelenk überbrückenden Gelenkschiene einer Coxarthrose-Gelenkorthese angeordnet und dort insbesondere ortsfest oder alternativ aktiv beweglich angeordnet.

Die distale Aktivpelotte kann also in einer ersten Variante ortsfest an dem Beckengurt selbst ausgebildet sein, so dass eine aktive Massagebewegung, vorzugsweise entlang der Längserstreckung der Weichteile in dieser Region, das heißt in proximal-distaler Richtung, allein durch die Auf- und Abbewegung der beteiligten Strukturen beim Gehen erzeugt wird.

Alternativ oder zusätzlich ist also vorgesehen, die distale Aktivpelotte unmittelbar mit oder an der Gelenkschiene einer Coxarthrose-Gelenkorthese zu verbinden, worin sie insbesondere über einen spezifischen Mechanismus in Abhängigkeit von der Auslenkung der Verschwenkung der Gelenkschiene aktiv geführt wird. Besonders ist vorgesehen, dass die distale Aktivpelotte eine primär abwärts gerichtete, das heißt nach distal gerichtete, Druck- und Zugwirkung auf den großen Rollhügel (Trochanter major) besonders am Ansatzpunkt des mittleren Gesäßmuskels, ausübt und dadurch eine Extension des Muskels und des Hüftgelenks bewirkt. Im synergistischen Zusammenspiel dieser distalen, optional aktiv bewegten, Aktivpelotte und des darüber liegenden, das heißt proximalen Noppenkissens mit mindestens einer vorstehenden Richtung mittleren Gesäßmuskels weisenden elastischen Noppe wird effizient eine aktive, einer aktiven Extensionsbehandlung in einer physikalischen Therapie bereits beim Tragen der Orthese bewirkt, sei es allein als Beckengurt, sei es als vollständige Coxarthrose-Gelenkorthese. Durch diese "eingebaute" manuelle Therapiefunktion wird der Tragekomfort der Orthese für den Patienten signifikant verbessert. Gleichzeitig wird zusätzlich zu der üblichen Führungs- und Stützfunktion einer Coxarthrose-Gelenkorthese eine therapeutische oder therapieunterstützende Wirkung erzielt. In einer Variante ist die distale Aktivpelotte an der Gelenkplatte des proximalen Arms der Gelenkschiene angeordnet. Dort ist vorgesehen, die distale Aktivpelotte in Abhängigkeit des Verschwenkwinkels des Gelenks der Gelenkschiene aktiv in proximal-distaler Richtung zu bewegen.

Der erfindungsgemäße Coxarthrose-Beckengurt ist einsetzbar ohne gelenkübergreifende stützende Gelenkschiene in Therapie und Rehabilitation von Beschwerden am Hüftgelenk eines Patienten, beispielsweise auch in Vorbereitung einer nachfolgenden Extensionsbehandlung im Rahmen einer physikalischen Therapie.

Ein Gegenstand gemäß des zweiten Aspekts der Erfindung ist eine Coxarthrose-Gelenkorthese als solche, worin der erfindungsgemäße Beckengurt mit einer hüftgelenküberbrückenden Gelenkschiene aufgerüstet ist, wobei die Gelenkschiene mit ihrem proximalen Arm über die Trägerplatte mit dem Beckengurt verbunden ist. Die erfindungsgemäße Gelenkorthese weist weiter eine Oberschenkelfassung auf, die ihrerseits mit dem distalen Arm dieser Gelenkschiene verbunden ist, um eine gelenküberbrückende Gelenkorthese zu bilden.

Schließlich ist ein weiterer Gegenstand der vorliegenden Offenbarung die Verwendung eines Noppenkissens mit mindestens einer elastischen Noppe mit bevorzugt feinstrukturierter Oberfläche, welche an einem Coxarthrose-Beckengurt gemäß der vorliegenden Erfindung ausgebildet ist, wobei die mindestens eine elastische Noppe so an dem Beckengurt positioniert ist, dass sie im angelegten Zustand des Beckengurts zu dem mittleren Gesäßmuskel weist, mit dem Zweck der Verbesserung des Tragekomforts einer Coxarthrose-Gelenkorthese. Eine alternative Verwendung ist die therapeutische Anwendung des Noppenkissens an dem Beckengurt zur Therapie von Beschwerden am Hüftgelenk eines Patienten, besonders der Coxarthrose, besonders sowohl im präoperativen als auch im postoperativen Zustand.

Die Erfindung wird nun anhand der in den nachfolgenden Figuren dargestellten Ausführungsvarianten näher beschrieben, ohne dass diese beschränkend zu verstehen wären.

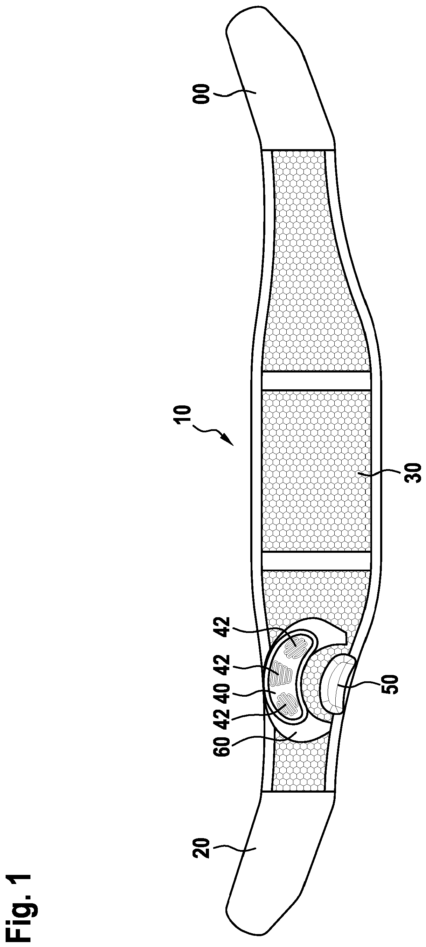

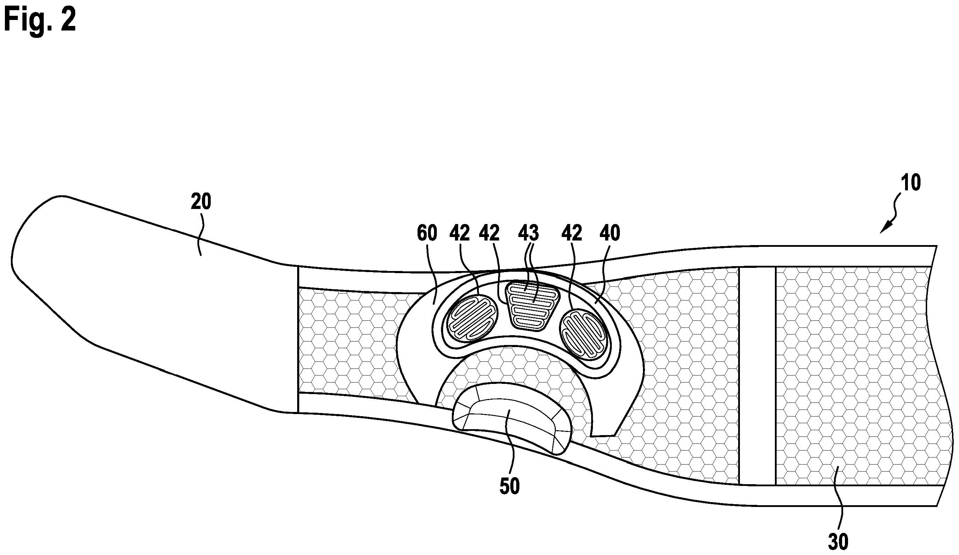

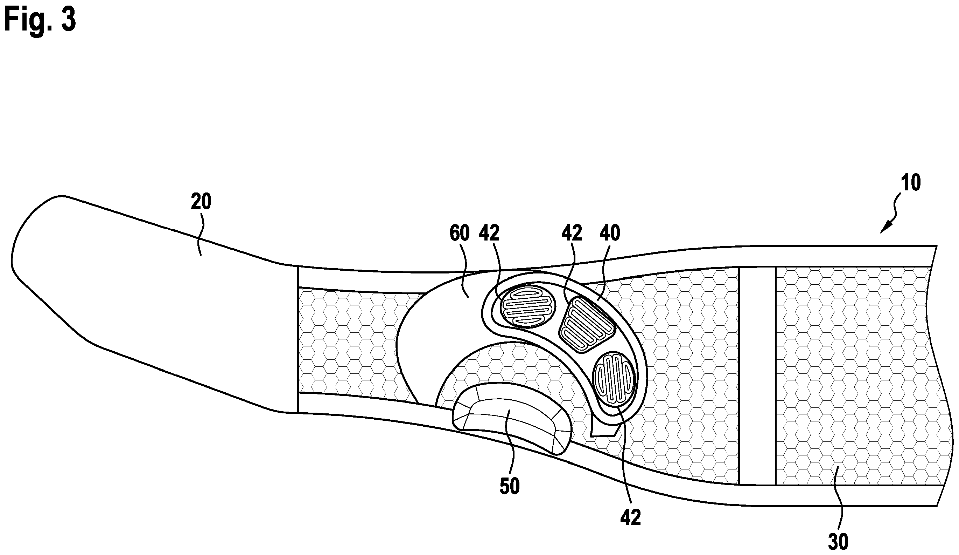

Figur 1 zeigt eine Gesamtansicht eines erfindungsgemäßen Beckengurts, geeignet zur alleinigen Verwendung als Coxarthrose-Bandage oder alternativ als Bestandteil einer Coxarthrose-Gelenkorthese. Der Beckengurt (10) weist einen Beckengurtgrundkörper (30) und zwei seitliche Gurtenden (20) auf, welche zum Schließen des Beckengurts unter Spannung in an sich bekannter Weise miteinander lösbar verkoppelbar sind. Der Beckengurt (10) ist in der hier dargestellten Ausführung aus einem teilelastischen Gestrick gebildet, um eine entsprechende Zugspannung an Becken und Hüfte des Patienten aufrechtzuerhalten. Erfindungsgemäß ist im Bereich zumindest eines Endes (20) des Beckengurts ein kreisbogenförmiges Noppenkissen (40) vorgesehen, was vorstehende Noppen (42) mit bevorzugt feinstrukturierter Oberseite (43) aufweist. Das Noppenkissen (40) weist im angelegten Zustand des Beckengurts (10) zu dem Patienten und ist erfindungsgemäß im Bereich seines Mittleren Gesäßmuskels, das heißt besonders proximal zu dem Hüftgelenk, positioniert. In der dargestellten Ausführung ist das Noppenkissen (40) an einer unelastischen, dennoch flexiblen Trägerplatte (60), die mit dem Beckengurtgrundkörper (30) fest verbunden ist, angeordnet und dort fixiert. Zusätzlich ist eine distale Aktivpelotte (50) optional unmittelbar an dem Beckengurtgrundkörper ausgebildet und dort fixiert. Figur 2 zeigt eine Detailansicht der Ausführung gemäß Figur 1. Das Noppenkissen (40) ist kreisbogenförmig ausgebildet und weist drei voneinander beabstandete vorstehende Noppen (42) mit bevorzugt feinstrukturierter Oberseite (43) auf. Wie Figur 3 ergänzend zu Figur 2 zeigt, ist bevorzugt bevorzugt vorgesehen, dass das Noppenkissen (40) an der Trägerplatte (60) ortsveränderlich fixierbar ist, besonders um die Noppen (42) bei Einrichtung des Beckengurts zur Verwendung als Orthese gezielt an "Triggerpunkte" des Mittleren Gesäßmuskels zu positionieren.

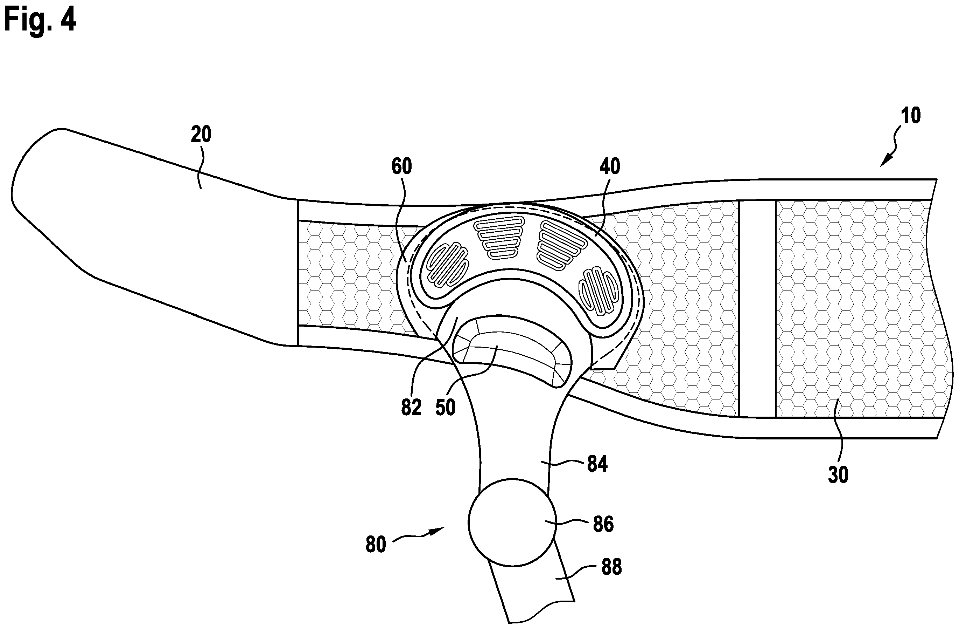

Figur 4 zeigt eine weitere Variante der Ausführung gemäß Figur 1 in Detailansicht, wobei in die Trägerplatte (60) zusätzlich die Gelenkplatte (82) eines proximalen Gelenkarms (84) einer Gelenkschiene (80) mit Gelenk (86) eingesetzt ist. In dieser Variante ist der Beckengurt (10) somit essenzieller Bestandteil einer Coxarthrose-Gelenkorthese. Dabei ist besonders vorgesehen, dass eine distale Aktivpelotte (50) an dem Gelenkarm (80) selbst, besonders an der oberen Gelenkplatte (82), ausgebildet und bevorzugt dort ortsfest oder beweglich fixiert ist.

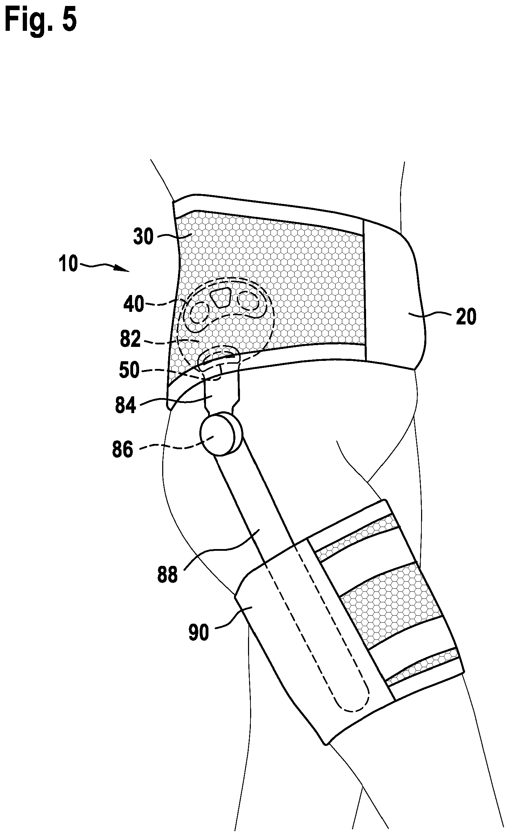

Figur 5 zeigt schließlich eine perspektivische Schrägansicht einer erfindungsgemäßen Coxarthrose-Gelenkorthese mit einem proximalen Beckengurt (10) gemäß der Erfindung, welcher mit einer Gelenkschiene (80) mit Axialgelenk (86) aufgerüstet ist, und einer mit dem distalen Arm (88) der Gelenkschiene (80) verbundenen distalen Oberschenkelfassung (90). In der Zeichnung angedeutet sind unterhalb der Gelenkplatte (82) des proximalen Arms (84) der Gelenkschiene (80) zum Körper des Patienten weisend das proximale Noppenkissen (40) und die distale Aktivpelotte (50) angeordnet.

## Patentansprüche

1. Coxarthrose-Beckengurt (10) mit einem Beckengurtgrundkörper (30) und zwei zum Anlegen des Beckengurts (10) um die Hüfte eines Patienten verbindbare seitliche Beckengurtenden (20), wobei an dem Beckengurt (10) an der Innenseite im Bereich des Beckengurtendes (20) mindestens ein Noppenkissen (40) mit mindestens zwei oder mehr sich davon erstreckender und zueinander beabstandet angeordneten Noppen (42) ausgebildet ist, wobei die Noppen (42) auf dem Noppenkissen (40) so positioniert sind, dass sie im angelegten Zustand des Beckengurts (10) zu dem Mittleren Gesäßmuskel des Patienten hin gerichtet sind, **dadurch gekennzeichnet, dass** die Noppen (42) an ihrer zu dem Muskel weisenden Oberseite mit mehreren schmalen Längsrippen, die im angelegten Zustand des Beckengurts (10) im Wesentlichen parallel zu dem Muskelfaserverlauf des Mittleren Gesäßmuskels orientiert sind, feinstrukturiert sind.

2. Beckengurt (10) nach Anspruch 1, wobei der Beckengurt (10) zusätzlich eine distale Aktivpelotte (50) aufweist und das Noppenkissen (40) an dem Beckengurt (10) zu der distalen Aktivpelotte (50) benachbart angeordnet ist.

3. Beckengurt (10) nach Anspruch 2, wobei das Noppenkissen (40) kreisbogenförmig um die distale Aktivpelotte (50) ausgebildet ist.

4. Beckengurt nach einem der vorstehenden Ansprüche, wobei auf dem Noppenkissen (40) mindestens zwei Noppen (42) zueinander beabstandet angeordnet sind.

5. Beckengurt (10) nach einem der vorstehenden Ansprüche, wobei der Beckengurtgrundkörper (30) eine unelastische Trägerplatte (60) aufweist, worauf das Noppenkissen (40) ortsveränderlich fixierbar ist.

6. Beckengurt (10) nach Anspruch 5, wobei über die Trägerplatte (60) eine Gelenkplatte (82) eines proximalen Arms (84) einer das Hüftgelenk überbrückenden Gelenkschiene (80) mit dem Beckengurtgrundkörper (30) verbindbar ist.

7. Beckengurt (10) nach Anspruch 6, wobei die distale Aktivpelotte (50) an der Gelenkplatte (82) angeordnet ist.

8. Coxarthrose-Gelenkorthese, enthaltend den Beckengurt (10) nach einem der Ansprüche 5 bis 7, eine das Hüftgelenk überbrückende Gelenkschiene (80), die mit ihrem proximalen Arm (84) über die Trägerplatte (60) mit dem Beckengurt (10) verbunden ist, und eine Oberschenkelfassung (90), die mit einem distalen Arm (88) der Gelenkschiene (80) verbunden ist.

## Claims

1. A coxarthrosis pelvic strap (10) comprising a pelvic strap base body (30) and two lateral pelvic strap ends (20) connectable for fitting the pelvic strap (10) around the hip of a patient, wherein at least one nub cushion (40) is formed on the pelvic strap (10) on the inside in the region of the pelvic strap end (20) with at least two or more nubs (42) extending therefrom and spaced apart from each other, wherein the nubs (42) are positioned on the nub cushion (40) such that they are directed towards the patient's medial gluteal muscle when the pelvic strap (10) is in the worn state, **characterised in that** the nubs (42) are finely structured on their upper side facing the muscle with a plurality of narrow longitudinal ribs which, when the pelvic strap (10) is in the worn state, are oriented substantially parallel to the course of the muscle fibres of the medial gluteal muscle.

2. The pelvic strap (10) according to claim 1, wherein the pelvic strap (10) additionally comprises a distal active pad (50) and the nub cushion (40) is arranged on the pelvic strap (10) adjacent to the distal active pad (50).

3. The pelvic strap (10) according to claim 2, wherein the nub cushion (40) is formed in a circular arc around the distal active pad (50).

4. The pelvic strap according to any one of the preceding claims, wherein at least two nubs (42) are spaced apart from each other on the nub cushion (40).

5. The pelvic strap (10) according to one of the preceding claims, wherein the pelvic strap base body (30) has an inelastic carrier plate (60), on which the nub cushion (40) can be fixed in a positionally variable manner.

6. The pelvic strap (10) according to claim 5, wherein a joint plate (82) of a proximal arm (84) of a hip joint bridging joint splint (80) can be connected to the pelvic strap base body (30) via the carrier plate (60).

7. The pelvic strap (10) according to claim 6, wherein the distal active pad (50) is arranged on the joint plate (82).

8. A coxarthrosis joint orthosis comprising the pelvic strap (10) of any one of claims 5 to 7, a hip joint bridging joint splint (80) having its proximal arm (84) connected to the pelvic strap (10) via the support plate (60), and a thigh socket (90) connected to a distal arm (88) of the joint splint (80).

## Revendications

1. Sangle de pelvienne pour coxarthrose (10) comprenant un corps de base de sangle de pelvienne (30) et deux bouts de sangle de pelvienne latéraux (20) pouvant être connectés pour ajuster la sangle de pelvienne (10) autour de la hanche d'un patient, dans laquelle au moins un coussin à ergots (40) est formé sur la sangle de pelvienne (10) sur l'intérieur dans la région du bout de sangle de pelvienne (20) avec au moins deux ergots ou plus (42) s'étendant à partir de celui-ci et espacés les uns des autres, les ergots (42) étant positionnés sur le coussin à ergots (40) de telle sorte qu'ils sont dirigés vers le muscle fessier médian du patient lorsque la sangle pelvienne (10) est à l'état usé, **caractérisé en ce que** les ergots (42) sont finement structurés sur leur côté supérieur tourné vers le muscle avec une pluralité de nervures longitudinales étroites qui, lorsque la sangle pelvienne (10) est à l'état usé, sont orientées substantiellement parallèlement au tracé des fibres musculaires du muscle fessier médian.

2. Sangle pelvienne (10) selon la revendication 1, dans laquelle la sangle pelvienne (10) comprend en outre un pad actif distal (50) et le coussin à ergots (40) est disposé sur la sangle pelvienne (10) de manière adjacente au pad actif distal (50).

3. Sangle pelvienne (10) selon la revendication 2, dans laquelle le coussin à ergots (40) est formé en arc de cercle autour du pad actif distal (50).

4. Sangle pelvienne selon l'une quelconque des revendications précédentes, dans laquelle au moins deux ergots (42) sont espacés l'un de l'autre sur le coussin à ergots (40).

5. Sangle pelvienne (10) selon l'une des revendications précédentes, dans laquelle le corps de base de la sangle pelvienne (30) a une plaque de support inélastique (60), sur laquelle le coussin à ergots (40) peut être fixé de manière variable en position.

6. Sangle pelvienne (10) selon la revendication 5, dans laquelle une plaque d'articulation (82) d'un bras proximal (84) d'une attelle articulaire de pontage de l'articulation de la hanche (80) peut être reliée au corps de base de la sangle pelvienne (30) par l'intermédiaire de la plaque de support (60).

7. Sangle pelvienne (10) selon la revendication 6, dans laquelle le pad actif distal (50) est disposé sur la plaque d'articulation (82).

8. Orthèse articulaire de coxarthrose comprenant la sangle pelvienne (10) de l'une quelconque des revendications 5 à 7, une attelle articulaire de pontage de l'articulation de la hanche (80) ayant son bras proximal (84) connecté à la sangle pelvienne (10) via la plaque de support (60), et une douille de cuisse (90) connectée à un bras distal (88) de l'attelle articulaire (80).
